# EUROPEAN PATENT SPECIFICATION

(11) **EP 4 272 789 B1**
(45) Date of publication and mention of the grant of the patent: **28.01.2026**
(21) Application number: 20967874.7
(22) Date of filing: 31.12.2020
(51) Int. Cl.: A61M 16/00, A61M 16/01, F04D 17/16, F04D 25/08, F04D 29/42, F04D 29/58, F04D 29/66

(54) **ANESTHESIA MACHINE WITH A DRIVING GAS CIRCUIT COMPRISING A FAN ASSEMBLY**
ANÄSTHESIEGERÄT MIT EINEM EINE GEBLÄSEANORDNUNG AUFWEISENDEN TREIBGASKREISLAUF
MACHINE D'ANESTHÉSIE AVEC UN CIRCUIT DE GAZ D'ENTRAÎNEMENT COMPRENANT UN ENSEMBLE VENTILATEUR

(43) Date of publication of application: 08.11.2023
(73) Proprietor: Shenzhen Mindray Animal Medical Technology Co., Ltd., Shenzhen, Guangdong 518110 (CN)
(72) Inventor: LI, Jicheng, Shenzhen, Guangdong 518057 (CN); CHEN, Yiwei, Shenzhen, Guangdong 518057 (CN)
(74) Representative: KIPA AB
(86) International application number: PCT/CN2020/142436
(87) International publication number: WO 2022/141536

(56) References cited:
- WO-A1-2008/098382
- WO-A1-2011/017763
- WO-A1-2020/087397
- CN-A- 100 998 902
- CN-A- 102 695 536
- CN-A- 104 394 920
- CN-A- 105 435 346
- CN-U- 208 097 087
- CN-U- 211 536 067
- FR-A3- 3 091 731
- US-A- 6 131 571
- US-A1- 2012 301 267
- US-A1- 2019 366 025
- US-A1- 2020 360 635

## Description

### TECHNICAL FIELD

The disclosure relates to the field of medical instruments, and in particular to an anesthesia machine, a veterinary anesthesia machine, and a fan assembly.

### BACKGROUND

A fan assembly of the existing anesthesia machine uses a heat sink or heat pipe to dissipate heat generated in operation of a motor to the outside, and uses a cooling fan to dissipate the heat forcedly. This heat dissipation structure is complex and costly.

In international patent application publication number WO 2020/087397 A1 an anesthesia respiration apparatus and method are disclosed.

In US patent application publication number US 2020/360635 A1 a ventilator system is presented.

In international patent application publication number WO 2011 /017763 A1 a blower is disclosed for a ventilator.

In US patent application publication number US 2019/366025A1 a blower is disclosed for respiratory gas.

In international patent application publication number WO 2008/098382 A1 an anesthetic machine is disclosed comprising a blower, which is provided with an electric motor and at least one compressor wheel, driven by the electric motor.

In Chinese patent application publication number CN 105435346A, a fan assembly and a ventilator using the same are disclosed.

In US patent application publication number US 2012/301267A1 a delivering unit for gas is disclosed comprising a compressor in a noise-insulation housing.

In French patent application publication number FR 3 091 731 A3 a ventilator is disclosed with a motor and a fan.

### SUMMARY OF THE INVENTION

The present invention provides an anesthesia machine as defined in claim 1. Further preferred embodiments of the present invention are defined in the dependent claims.

### SUMMARY OF THE DISCLOSURE

In view of this, the disclosure provides an anesthesia machine, a veterinary anesthesia machine, and a fan assembly.

In a first aspect of the disclosure, provided is an anesthesia machine, including a driving gas branch, a fresh gas branch, and a breathing circuit, where the fresh gas branch is configured to deliver fresh gas with anesthetic gas into the breathing circuit, the driving gas branch is configured to push the fresh gas from the breathing circuit to a patient, the driving gas branch includes a fan assembly, the fan assembly includes a housing, a fan and a first heat-dissipating member, the housing includes a first inner cavity, a first air inlet communicating with the first inner cavity, and a first air outlet communicating with the first inner cavity, the fan is disposed in the first inner cavity, the fan is configured to drive air from the first air inlet into the first inner cavity and blow the air out from the first air outlet, and the fan includes:
a volute;
an impeller, rotatably mounted in the volute; and
a motor, mounted in the volute and connected to the impeller, and configured to drive the impeller to rotate;
where the first heat-dissipating member is mounted on the motor and connected to the housing, and the first heat-dissipating member is configured to conduct heat generated by the motor to the housing.

In a second aspect of the disclosure, provided is a veterinary anesthesia machine, including a driving gas branch, a fresh gas branch, and a breathing circuit, where the fresh gas branch is configured to deliver fresh gas with anesthetic gas into the breathing circuit, the driving gas branch is configured to push the fresh gas from the breathing circuit to a target object, the driving gas branch includes a fan assembly, the fan assembly includes a housing, a fan, and a first heat-dissipating member, the housing includes a first inner cavity, a first air inlet communicating with the first inner cavity, and a first air outlet communicating with the first inner cavity, the fan is disposed in the first inner cavity, the fan is configured to drive air from the first air inlet into the first inner cavity and blow the air out from the first air outlet, and the fan includes:
a volute;
an impeller, rotatably mounted in the volute; and
a motor, mounted in the volute and connected to the impeller, and configured to drive the impeller to rotate;
where the first heat-dissipating member is mounted on the motor and connected to the housing, and the first heat-dissipating member is configured to conduct heat generated by the motor to the housing.

In a third aspect of the disclosure, provided is a fan assembly, including a housing, a fan, and a first heat-dissipating member, the housing includes a first inner cavity, a first air inlet communicating with the first inner cavity, and a first air outlet communicating with the first inner cavity, the fan is disposed in the first inner cavity, the fan is configured to drive air from the first air inlet into the first inner cavity and blow the air out from the first air outlet, and the fan includes:
a volute;
an impeller, rotatably mounted in the volute; and
a motor, mounted in the volute and connected to the impeller, and configured to drive the impeller to rotate;
where the first heat-dissipating member is mounted on the motor and connected to the housing, and the first heat-dissipating member is configured to conduct heat generated by the motor to the housing.

It can be seen from the above technical solutions that in the anesthesia machine proposed in the first aspect of the disclosure, the first heat-dissipating member is provided to conduct the heat generated by the motor to the housing and then dissipate the heat from the housing, so as to cool the motor. This heat dissipation structure is simple and low in cost.

### BRIEF DESCRIPTION OF THE DRAWINGS

In order to more clearly describe the technical solutions in the embodiments of the disclosure, the drawings required for describing the embodiments will be briefly described below. Apparently, the drawings in the following description show some of the embodiments of the disclosure, and those of ordinary skill in the art may still derive other drawings from these drawings without creative efforts.
FIG. 1 is a schematic block diagram of a partial structure of an anesthesia machine according to an embodiment of the disclosure;
FIG. 2 is a connection schematic diagram of a driving gas branch, a fresh gas branch and a breathing circuit of an anesthesia machine according to an embodiment of the disclosure;
FIG. 3 is a schematic structural diagram a fan assembly according to an embodiment of the disclosure;
FIG. 4 is a schematic exploded diagram of a fan assembly according to an embodiment of the disclosure in a first view;
FIG. 5 is a schematic exploded diagram of a fan assembly according to an embodiment of the disclosure in a second view;
FIG. 6 is a schematic structural diagram of a first heat-dissipating member according to an embodiment of the disclosure;
FIG. 7 is a schematic structural diagram of a first elastic supporting member according to an embodiment of the disclosure; and
FIG. 8 is a schematic structural diagram of a second elastic supporting member according to an embodiment of the disclosure.

### DESCRIPTION OF THE PREFERRED EMBODIMENTS

The technical solutions of the embodiments of the disclosure will be described below clearly and comprehensively in conjunction with accompanying drawings of the embodiments of the disclosure. Apparently, the embodiments described are some of, rather than all of, the embodiments of the disclosure. All other embodiments obtained by persons of ordinary skill in the art based on the embodiments of the disclosure without creative efforts shall fall within the scope of protection of the disclosure.

It should also be understood that the terms used in the description of the disclosure are only intended to describe specific embodiments, but not to limit the disclosure. As used in the description and the appended claims, unless the context clearly indicates otherwise, the singular forms "a", "an" and "the" are intended to include the plural forms.

It should also be further understood that the term "and/or" used in the description and the appended claims refers to one of the items listed correlatively or any combination and all possible combinations of the items, and includes these combinations.

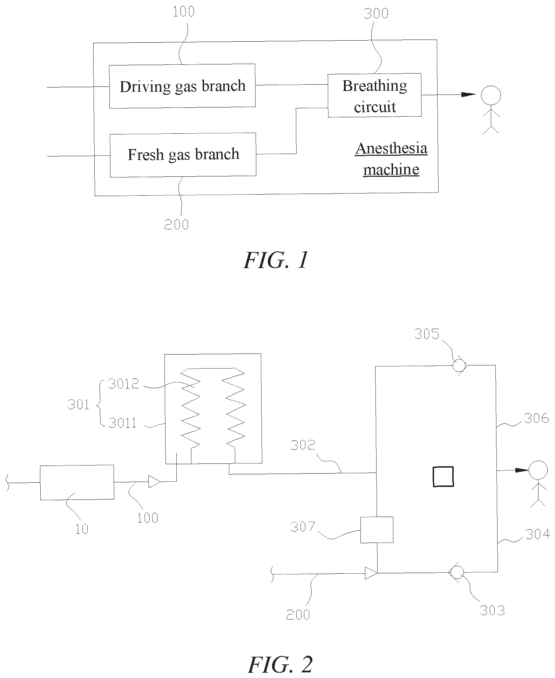

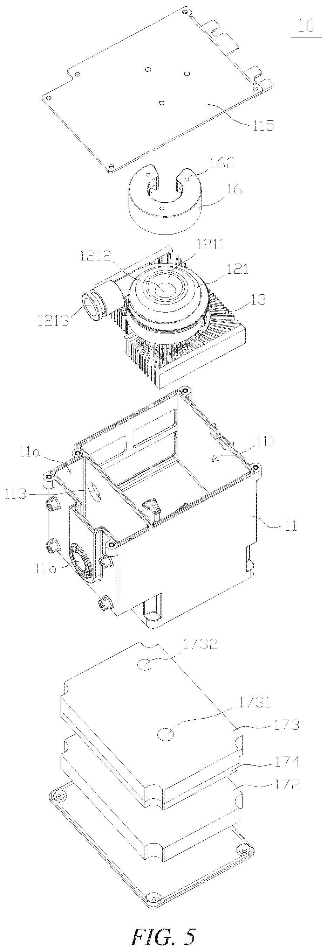

As shown in FIG. 1 to FIG. 5, an embodiment of the disclosure provides an anesthesia machine, including a driving gas branch 100, a fresh gas branch 200, and a breathing circuit 300. The fresh gas branch 200 is configured to deliver fresh gas with anesthetic gas into the breathing circuit 300. The driving gas branch 100 is configured to push the fresh gas from the breathing circuit 300 to a patient. The driving gas branch 100 includes a fan assembly 10. The fan assembly 10 includes a housing 11 and a fan 12. The housing 11 includes a first inner cavity 111, a first air inlet 112 communicating with the first inner cavity 111, and a first air outlet 113 communicating with the first inner cavity 111. The fan 12 is disposed in the first inner cavity 111. The fan 12 is configured to drive air from the first air inlet 112 into the first inner cavity 111 and blow the air out from the first air outlet 113. The fan 12 includes a volute 121, an impeller (not shown in the figures) and a motor 123, the impeller is rotatably mounted in the volute 121, and the motor 123 is mounted in the volute 121 and connected to the impeller, and is configured to drive the impeller to rotate. The first air inlet 112 is located close to the motor 123 so that most of air entering the first inner cavity 111 from the first air inlet 112 flows over a surface of the motor 123 to carry away heat generated in operation of the motor 123.

Optionally, the first air inlet 112 is opposite to the motor 123, and the opposite arrangement not only includes the first air inlet 112 and the motor 123 being directly opposite each other in an air intake direction of the first air inlet 112, but also includes the first air inlet 112 and the motor 123 partially overlapping in the air intake direction of the first air inlet 112, provided that an airflow entering the first inner cavity 111 from the first air inlet 112 can be blown to the motor 123.

By way of example, FIG. 2 shows a connection relationship of the driving gas branch 100, the fresh gas branch 200 and the breathing circuit 300 in an embodiment. The breathing circuit 300 includes a mechanically controlled driving assembly 301, a main pipeline 302, an inhalation check valve 303, a gas supply pipeline 304, an exhalation check valve 305, a gas return pipeline 306 and a carbon dioxide absorption tank 307. The gas supply pipeline 304 is connected to one end of the main pipeline 302, and the inhalation check valve 303 and the carbon dioxide absorption tank 307 are mounted in the gas supply pipeline 304. The gas return pipeline 306 connects the gas supply pipeline 304 to the main pipeline 302, and the exhalation check valve 305 is mounted in the gas return pipeline 306. The mechanically controlled driving assembly 301 includes an air bellow 3011 and a folding airbag 3012. The folding airbag 3012 communicates with the other end of the main pipeline 302. The driving gas branch 100 communicates with a cavity enclosed by the air bellow 3011 and the folding airbag 3012. The fresh gas branch 200 is connected between the inhalation check valve 303 and the carbon dioxide absorption tank 307. During operation, gas that a patient exhales enters the folding airbag 3012 through the exhalation check valve 305 and the main pipeline 302, the driving gas branch 100 provides a driving gas into the cavity enclosed by the air bellow 3011 and the folding airbag 3012, and the driving gas compresses the folding airbag 3012, so that the gas that the patient exhales is delivered to the carbon dioxide absorption tank 307 through the main pipeline 302, the carbon dioxide absorption tank 307 absorbs carbon dioxide in the exhaled gas, and gas discharged from the carbon dioxide absorption tank 307 is mixed with the fresh gas supplied by the fresh gas branch 200 and then delivered to the patient through the gas supply pipeline 304.

In the anesthesia machine proposed in this embodiment, since the first air inlet 112 is disposed opposite the motor 123, the airflow entering the first inner cavity 111 from the first air inlet 112 to the motor 123 can carry away the heat generated in operation of the motor 123 so as to cool the motor 123. This embodiment ingeniously utilizes the structural design of the fan assembly 10 itself, and this heat dissipation structure is simple and low in cost.

Optionally, the volute 121 includes a second inner cavity 1211, a second air inlet 1212 communicating with the second inner cavity 1211, and a second air outlet 1213 communicating with the second inner cavity 1211. The impeller is disposed in the second inner cavity 1211, the second air outlet 1213 communicates with the first air outlet 113, and the second air inlet 1212 faces away from the first air inlet 112.

Since the second air inlet 1212 faces away from the first air inlet 112, air entering the first inner cavity 111 from the first air inlet 112 will pass through an outer side wall of the volute 121 after passing through the motor 123 and before entering the second air inlet 1212, thus carrying away the heat transferred from the motor 123 to the volute 121 and further cooling the motor 123. Of course, the second air inlet 1212 is not limited to being disposed on the side facing away from the first air inlet 112, but can be disposed in other positions of the volute 121, which depends on the actual design needs.

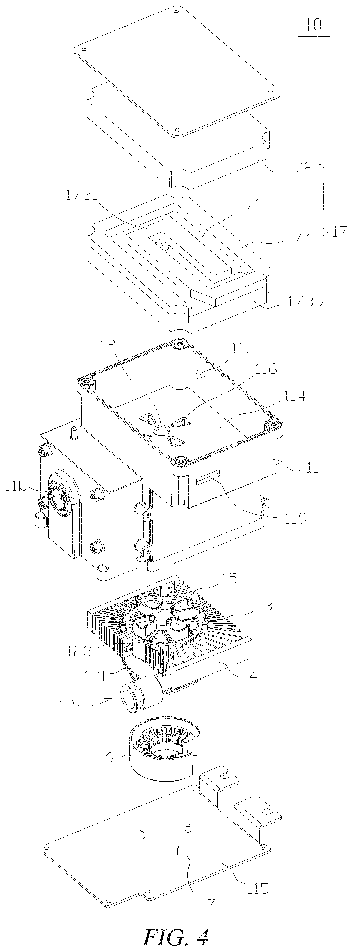

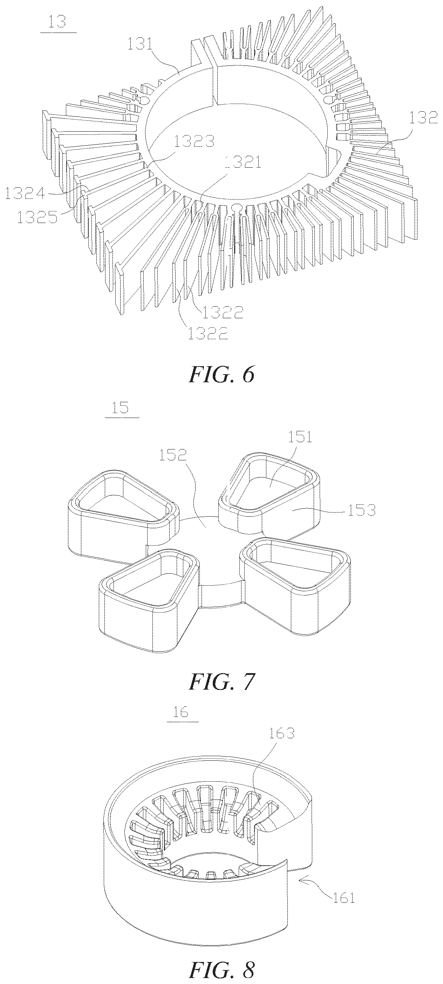

Optionally, as shown in FIG. 4 and FIG. 6, the fan assembly 10 further includes a first heat-dissipating member 13 mounted on the motor 123. The first heat-dissipating member 13 may have an effect of accelerating the heat dissipation of the motor 123 and is simple in structure and low in cost.

Optionally, the first heat-dissipating member 13 includes a connecting portion 131 and radiating fins 132, the connecting portion 131 is connected to the motor 123, and the radiating fins 132 are connected to the connecting portion 131 to expand a heat dissipation area. Of course, the first heat-dissipating member 13 may also be not provided with the radiating fins 132.

Optionally, the connecting portion 131 and the radiating fins 132 are formed integrally, for example, by casting.

Optionally, the first heat-dissipating member 13 is made of a copper-based or aluminum-based metal material, which has good thermal conductivity and can cool the motor 123 well at a low cost.

Optionally, the connecting portion 131 is in the form of a ring, the connecting portion 131 is sleeved over the motor 123, a plurality of radiating fins 132 are provided, and the plurality of radiating fins 132 are spaced around an outer side wall of the connecting portion 131.

Since the connecting portion 131 is sleeved over the motor 123, the motor 123 can be evenly cooled with a good heat dissipation effect. Of course, the connecting portion 131 is not limited to a ring shape, but can also be in other shapes, as long as the connecting portion 131 connects the motor 123 and the radiating fins 132 to cool the motor 123.

Optionally, at least one radiating fin 132 includes a fixed portion 1321 and two radiating sub-fins 1322. One end of the fixed portion 1321 is connected to the connecting portion 131 and the other end of the fixed portion 1321 is connected to the two radiating sub-fins 1322. This embodiment can further achieve the effect of expanding the heat dissipation area and accelerating the heat dissipation of the motor 123.

Optionally, the fan assembly 10 further includes a second heat-dissipating member 14, the second heat-dissipating member 14 is arranged between the first heat-dissipating member 13 and the housing 11, and the second heat-dissipating member 14 is configured to conduct the heat from the first heat-dissipating member 13 to the housing 11 and dissipate the heat from the housing 11.

Optionally, the second heat-dissipating member 14 may be made of a flexible material, such as one of a thermally conductive paste, a thermally conductive adhesive, a thermally conductive pad and a liquid metal. Preferably, the second heat-dissipating member 14 is made of a flexible material, for example, the second heat-dissipating member 14 is a flexible thermally conductive pad, the flexible thermally conductive pad can weaken the vibration of the motor 123 transmitted to the housing 11 via the first heat-dissipating member 13, thus reducing the generation of noise. Further, the first heat-dissipating member 13 may also be made of a flexible material, which can effectively prevent the vibration of the motor 123 from being transmitted to the housing 11.

Optionally, the housing 11 is made of a metal material. The metal material has good thermal conductivity and can quickly conduct the heat generated by the motor 123 to the housing 11 and dissipate the heat from the housing 11. Available metal materials include a copper-based or aluminum-based metal material. Of course, the housing 11 is not limited to being made of a metal material, for example, the housing 11 may also be made of thermally conductive plastic. Available thermally conductive plastics include PP (polypropylene), ABS (Acrylonitrile Butadiene Styrene plastic), PC (Polycarbonate), PA (Nylon Polyamide), LCP (Liquid Crystal Polymer), PPS (Phenylenesulfide), and PEEK (Peek materials).

It should be noted that it is not necessary that the entire housing 11 is made of a metal material or thermally conductive plastic. In some embodiments, only part of the first heat-dissipating member 13 is in contact with the housing 11 via the second heat-dissipating member 14. In this embodiment, only the side wall of the housing 11 that is in contact with the second heat-dissipating member 14 is made of a metal material or thermally conductive plastic.

Optionally, the radiating fins 132 each include a first end 1323 and a second end 1324, the first end 1323 is connected to the connecting portion 131, the second ends 1324 of at least some of the radiating fins 132 are provided with contact portions 1325 at included angles to the radiating fins 132, a plurality of contact portions 1325 are arranged in the same plane, or a plurality of contact portions 1325 are connected to form a contact plate, and the second heat-radiating member 14 is sandwiched between the contact portions 1325 and the housing 11.

The contact portions 1325 are provided, and the plurality of contact portions 1325 are arranged in the same plane or connected to form the contact plate, so the connection between the radiating fins 132 and the housing 11 is facilitated, and the contact area between the radiating fins 132 and the housing 11 can also be expanded, thus accelerating the conduction of heat from the radiating fins 132 to the housing 11.

As shown in FIG. 4, FIG. 5, FIG. 7 and FIG. 8, optionally, the fan assembly 10 further includes a first elastic supporting member 15 and a second elastic supporting member 16, the housing 11 includes a top plate 114 and a bottom plate 115 opposite the top plate 114, the first elastic supporting member 15 is sandwiched between the top plate 114 and the top of the fan 123, and the second elastic supporting member 16 is sandwiched between the bottom plate 115 and the bottom of the fan 123. The first elastic supporting member 15 and the second elastic supporting member 16 may function in damping to prevent the vibration of the fan 123 during operation from being transmitted directly to the housing 11 to result in noise.

Optionally, the first elastic supporting member 15 and the second elastic supporting member 16 are made of a silicone material.

Optionally, the top plate 114 of the housing 11 is provided with a mounting portion 116 protruding towards the first inner cavity 111, the first elastic supporting member 15 is provided with a mounting recess 151 matching the mounting portion 116 in shape, the first elastic supporting member 15 is mounted to the top plate 114 by embedding the mounting portion 116 into the mounting recess 151, and the top of the fan 123 abuts against the first elastic supporting member 15.

Exemplarily, four mounting portions 116 are provided, and the four mounting portions are equally spaced in a circular pattern. The first elastic supporting member 15 includes a body 152 and four parts 153 equally spaced around the body 152, each part 153 is provided with a mounting recess 151, and the four mounting portions 116 are embedded in the four parts 153, respectively.

Optionally, the second elastic supporting member 16 is disposed around the second air inlet 1212, and a side wall of the second elastic supporting member 16 is provided with a gap 161 communicating with the second air inlet 1212. Gas enters the second air inlet 1212 through the gap 161 and enters the second inner cavity 1211 through the second air inlet 1212. In some other embodiments, the side wall of the second elastic supporting member 16 may not be provided with the gap 161, for example, the second elastic supporting member 16 may be disposed apart from the bottom plate 115, and the gas enters the second air inlet 1212 from the side of the second elastic supporting member 16 facing the bottom plate 115.

Optionally, the bottom plate 115 of the housing 11 is provided with a mounting post 117 in a protruding manner, the second elastic supporting member 16 is provided with a mounting hole 162 at the bottom, the second elastic supporting member 16 is mounted to the bottom plate 115 by embedding the mounting post 117 into the mounting hole 162, and the bottom of the fan 123 abuts against the second elastic supporting member 16. Exemplarily, three mounting posts 117 are provided, three mounting holes 162 are provided, and the three mounting posts 117 are embedded into the three mounting holes 162, respectively.

Optionally, an inner side wall of the second elastic supporting member 16 is provided with a plurality of supporting ribs 163 spaced apart from each other, the bottom of the fan 123 abuts against the supporting ribs 163, and the supporting ribs 163 can reduce the contact area between the fan 123 and the second elastic supporting member 16, thus reducing the vibration of the fan 123 transmitted to the bottom plate 115 through the second elastic supporting member 16.

Optionally, the anesthesia machine further includes an air fan (not shown in the figures) disposed outside the housing 11. The air fan is configured to generate an airflow blowing towards the housing 11, and the airflow may carry away the heat from the housing 11 and accelerate the heat dissipation of the housing 11, thereby accelerating the heat dissipation of the motor 123.

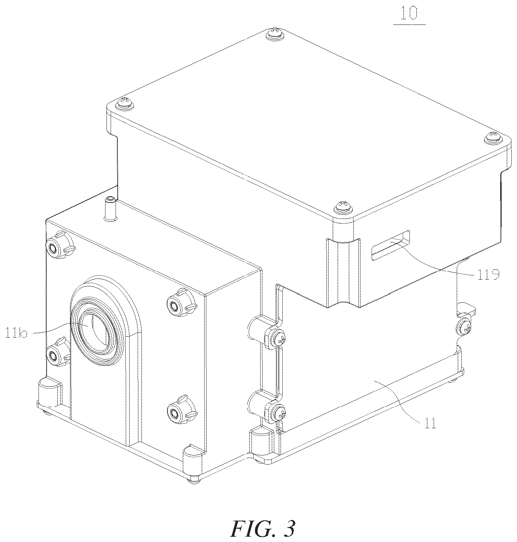

Optionally, as shown in FIG. 3 to FIG. 5, the housing 11 further includes a third inner cavity 118 and a third air inlet 119 communicating with the third inner cavity 118, and the third inner cavity 118 communicates with the first inner cavity 111 via the first air inlet 112. The fan assembly 10 further includes a first noise reduction assembly 17 disposed in the third inner cavity 118, the first noise reduction assembly 17 being configured to reduce noise of the fan from the third air inlet 119.

Since the first noise reduction assembly 17 is disposed in the third inner cavity 118 to reduce noise of the fan from the third air inlet 119, the impact of noise on the patient can be effectively reduced.

Optionally, the first noise reduction assembly 17 is provided with a noise reduction channel 171, one end of the noise reduction channel 171 communicating with the first air inlet 112 and the other end of the noise reduction channel 171 communicating with the third air inlet 119. On the one hand, the noise reduction channel 171 is configured to connect the first air inlet 112 to the third air inlet 119 so that air outside the fan assembly 10 can enter the first inner cavity 111 through the third air inlet 119, the noise reduction channel 171 and the first air inlet 112. On the other hand, the noise reduction channel 171 is configured to reduce the noise generated in operation of the fan 123 from spreading out of the third air inlet 119.

Optionally, the noise reduction channel 171 is spiral. The noise in the spiral noise reduction channel 171 can be well absorbed by the first noise reduction assembly 17 after multiple turns, thereby achieving a better attenuation effect on the noise. Of course, the noise reduction channel 171 is not limited to be spiral. For example, the noise reduction channel 171 may also be serpentine, L-shaped, linear, or honeycomb-shaped, which depends on the actual design needs.

Optionally, the first noise reduction assembly 17 includes an upper clamp plate 172, a lower clamp plate 173, and a spiral structural member 174 sandwiched between the upper clamp plate 172 and the lower clamp plate 173. The upper clamp plate 172, the lower clamp plate 173, and the spiral structural member 174 enclose to form the noise reduction channel 171, and the lower clamp plate 173 is provided with an opening 1731 connecting the noise reduction channel 171 to the first air inlet 112. The first noise reduction assembly 17 composed of the upper clamp plate 172, the lower clamp plate 173 and the spiral structural member 174 is easy to manufacture and assemble.

Optionally, the first noise reduction assembly 17 is made of a porous foam material. Exemplarily, the porous foam material may be acoustic foam, which has a good sound absorption effect and is low in cost.

Optionally, one of the first noise reduction assembly 17 and the top plate 114 is provided with a mistake-proofing hole 1732, and the other of the first noise reduction assembly 17 and the top plate 114 is provided with a mistake-proofing post (not shown in the figures) that penetrates the mistake-proofing hole 1732. The matching between the mistake-proofing post and the mistake-proofing hole 1732 facilitates assembling of the first noise reduction assembly 17, which can effectively prevent the first noise reduction assembly 17 from blocking the first air inlet 112 and the third air inlet 119 due to incorrect mounting of the first noise reduction assembly 17.

Optionally, the housing 11 further includes a fourth inner cavity 11a and a third air outlet 11b communicating with the fourth inner cavity 11a, and the fourth inner cavity 11a communicates with the first inner cavity 111 via the first air outlet 113.

Optionally, a second noise reduction assembly is disposed in the fourth inner cavity 11a. The second noise reduction assembly is configured to reduce noise of the fan from the third air outlet 11b. The second noise reduction assembly can be arranged in the same way as the first noise reduction assembly 17, which will not be described herein again.

Optionally, an inner side wall of the first inner cavity 111 is provided with noise reduction sponge to further reduce the noise in operation of the fan 123.

As shown in FIG. 1 to FIG. 5, an embodiment of the disclosure further provides a veterinary anesthesia machine, including a driving gas branch 100, a fresh gas branch 200, and a breathing circuit 300. The fresh gas branch 200 is configured to deliver fresh gas with anesthetic gas into the breathing circuit 300. The driving gas branch 100 is configured to push the fresh gas from the breathing circuit 300 to a patient. The driving gas branch 100 includes a fan assembly 10. The fan assembly 10 includes a housing 11, a fan 12, and a first heat-dissipating member 13. The housing 11 includes a first inner cavity 111, a first air inlet 112 communicating with the first inner cavity 111, and a first air outlet 113 communicating with the first inner cavity 111. The fan 12 is disposed in the first inner cavity 111. The fan 12 is configured to drive air from the first air inlet 112 into the first inner cavity 111 and blow the air out from the first air outlet 113. The fan 12 includes a volute 121, an impeller (not shown in the figures) and a motor 123, the impeller is rotatably mounted in the volute 121, and the motor 123 is mounted in the volute 121 and connected to the impeller, and is configured to drive the impeller to rotate. The first heat-dissipating member 13 is mounted to the motor 123 and connected to the housing 11, and the first heat-dissipating member 13 is configured to conduct heat generated by the motor 123 to the housing 11.

The above descriptions are merely the specific embodiments of the disclosure, but the scope of the disclosure is not limited thereto, those skilled in the art would readily think of various equivalent modifications or substitutions within the technical scope disclosed in the disclosure, and these modifications or substitutions should all be intended to be included within the scope of the disclosure.

The scope of the present invention is defined by the claims that follow.

## Claims

1. An anesthesia machine, wherein the anesthesia machine comprises a driving gas branch (100), a fresh gas branch (200), and a breathing circuit (300), wherein the fresh gas branch (200) is configured to deliver fresh gas with anesthetic gas into the breathing circuit (300), the driving gas branch (100) is configured to push the fresh gas from the breathing circuit (300) to a patient, the driving gas branch (100) comprises a fan assembly (10), **characterized in that**
the fan assembly (10) comprises a housing (11) , a fan (12), and a first heat-dissipating member (13),
the housing (11) comprises a first inner cavity (111), a first air inlet (112) communicating with the first inner cavity (111), and a first air outlet (113) communicating with the first inner cavity (111),
the fan (12) is disposed in the first inner cavity (111) and configured to drive air from the first air inlet (112) into the first inner cavity (111) and blow the air out from the first air outlet (113), and
the fan (12) comprises:
a volute (121);
an impeller, rotatably mounted in the volute (121); and
a motor (123), mounted in the volute (121) and connected to the impeller, and configured to drive the impeller to rotate;
wherein the first heat-dissipating member (13) is mounted on the motor (123) and connected to the housing (11), and the first heat-dissipating member (13) is configured to conduct heat generated by the motor (123) to the housing (11).

2. The anesthesia machine of claim 1, **characterized in that** the first heat-dissipating member (13) comprises:
a connecting portion (131) connected to the motor (123); and
radiating fins (132) connected to the connecting portion (131) to expand a heat dissipation area.

3. The anesthesia machine of claim 2, **characterized in that** the connecting portion (131) is in the form of a ring, the connecting portion is sleeved over the motor (123), a plurality of radiating fins are provided, and the plurality of radiating (132) fins are spaced around an outer side wall of the connecting portion.

4. The anesthesia machine of claim 3, **characterized in that** the fan assembly (10) further comprises a second heat-dissipating member (14), the second heat-dissipating member (14) is arranged between the first heat-dissipating member (13) and the housing (11), and the second heat-dissipating member (14) is configured to direct the heat from the first heat-dissipating member (13) to the housing.

5. The anesthesia machine of claim 4, **characterized in that** the radiating fins (132) each comprise a first end and a second end, the first end is connected to the connecting portion (131), the second ends of at least some of the radiating fins (132) are provided with contact portions (1325) at included angles to the radiating fins (132), a plurality of contact portions (1325) are arranged in the same plane, or a plurality of contact portions (1325) are connected to form a contact plate, and the second heat-radiating member (14) is sandwiched between the contact portions (1325) and the housing (11).

6. The anesthesia machine of claim 4, **characterized in that** the housing (11) is made of a metal material; or
the housing (11) is made of thermally conductive plastic; or
a side wall of the housing (11) that is in contact with the second heat-dissipating member (14) is made of a metal material or thermally conductive plastic.

7. The anesthesia machine of claim 1-6, **characterized in that** the first air inlet (112) is located close to the motor (123) so that most of gas entering the first inner cavity (111) from the first air inlet (112) flows over a surface of the motor (123) to carry away the heat generated in operation of the motor (123).

8. The anesthesia machine of claim 1-7, **characterized in that** the volute (121) comprises a second inner cavity (1211), a second air inlet (1212) communicating with the second inner cavity (1211), and a second air outlet (1213) communicating with the second inner cavity (1211), the impeller is disposed in the second inner cavity (1211), the second air outlet (1213) communicates with the first air outlet (113), and the second air inlet (1212) is provided close to the first air inlet (112).

9. The anesthesia machine of claim 1-8, **characterized in that** the fan assembly (10) further comprises a first elastic supporting member (15) and a second elastic supporting member (16), the housing (11) comprises a top plate (114) and a bottom plate (115) opposite the top plate (114), the first elastic supporting member (15) is sandwiched between the top plate (114) and the top of the fan, and the second elastic supporting member (16)is sandwiched between the bottom plate (115) and the bottom of the fan.

10. The anesthesia machine of claim 9, **characterized in that** the second elastic supporting member (16) is disposed around the second air inlet (1212), and a side wall of the second elastic supporting member (16) is provided with a gap communicating with the second air inlet (1212).

11. The anesthesia machine of claim 4, **characterized in that** the first heat-dissipating member (13) is connected to the housing (11) by the second heat-dissipating member (14), and at least one of the first heat-dissipating member (13) and the second heat-dissipating member (14) is made of a flexible material.

12. The anesthesia machine of claim 1, **characterized in that** the anesthesia machine further comprises
the housing (11) further comprising a third inner cavity (118), and a third air inlet (119) communicating with the third inner cavity (118), the third inner cavity (118) communicating with the first inner cavity (111) via the first air inlet (112);
the fan, disposed in the first inner cavity (111) and configured to drive air from the third air inlet (119) into the first inner cavity (111) and blow the air out from the first air outlet (113); and
a first noise reduction assembly (17), disposed in the third inner cavity (118) and configured to reduce noise of the fan from the third air inlet (119).

13. The anesthesia machine of claim 12, **characterized in that** the first noise reduction assembly (17) is provided with a noise reduction channel (171) , one end of the noise reduction channel (171) communicating with the first air inlet and the other end of the noise reduction channel (171) communicating with the third air inlet (119).

14. The anesthesia machine of claim 1-13, **characterized in that** the first air inlet is opposite to the motor (123).

15. The anesthesia machine of claim 14, **characterized in that** the first air inlet (112) is opposite to the motor (123) including (a) the first air inlet (112) and the motor (123) being directly opposite each other in an air intake direction of the first air inlet (112) or (b) the first air inlet (112) and the motor (123) partially overlapping in the air intake direction of the first air inlet (112).

## Patentansprüche

1. Narkosegerät, wobei das Narkosegerät einen Treibgaszweig (100), einen Frischgaszweig (200) und einen Beatmungskreislauf (300) umfasst, wobei der Frischgaszweig (200) dazu ausgestaltet ist, Frischgas mit Narkosegas in den Beatmungskreislauf (300) einzuführen, der Treibgaszweig (100) dazu ausgestaltet ist, das Frischgas von dem Beatmungskreislauf (300) zu einem Patienten zu drücken, und der Treibgaszweig (100) eine Gebläsebaugruppe (10) umfasst, **dadurch gekennzeichnet, dass**
die Gebläsebaugruppe (10) ein Gehäuse (11), ein Gebläse (12) und ein erstes Wärmeabführungsglied (13) umfasst,
das Gehäuse (11) einen ersten inneren Hohlraum (111), einen ersten Lufteinlass (112), der mit dem ersten inneren Hohlraum (111) kommuniziert, und einen ersten Luftauslass (113), der mit dem ersten inneren Hohlraum (111) kommuniziert, umfasst,
das Gebläse (12) in dem ersten inneren Hohlraum (111) angeordnet und dazu ausgestaltet ist, Luft von dem ersten Lufteinlass (112) in den ersten inneren Hohlraum (111) zu treiben und die Luft aus dem ersten Luftauslass (113) auszublasen, und
das Gebläse (12) Folgendes umfasst:
eine Schnecke (121),
ein drehbar in der Schnecke (121) montiertes Laufrad und
einen Motor (123), der in der Schnecke (121) montiert und mit dem Laufrad verbunden und dazu ausgestaltet ist, das Laufrad drehanzutreiben,
wobei das erste Wärmeabführungsglied (13) an dem Motor (123) montiert und mit dem Gehäuse (11) verbunden ist und das erste Wärmeabführungsglied (13) dazu ausgestaltet ist, von dem Motor (123) erzeugte Wärme zu dem Gehäuse (11) zu leiten.

2. Narkosegerät nach Anspruch 1, **dadurch gekennzeichnet, dass** das erste Wärmeabführungsglied (13) Folgendes umfasst:
einen mit dem Motor (123) verbundenen Verbindungsabschnitt (131) und
strahlenförmige Rippen (132), die mit dem Verbindungsabschnitt (131) verbunden sind, um einen Wärmeabführungsbereich zu expandieren.

3. Narkosegerät nach Anspruch 2, **dadurch gekennzeichnet, dass** der Verbindungsabschnitt (131) in der Form eines Rings ist, der Verbindungsabschnitt auf den Motor (123) aufgeschoben ist, eine Vielzahl von strahlenförmigen Rippen vorgesehen sind und die Vielzahl von strahlenförmigen Rippen um eine äußere Seitenwand des Verbindungsabschnitts herum beabstandet sind.

4. Narkosegerät nach Anspruch 3, **dadurch gekennzeichnet, dass** die Gebläsebaugruppe (10) ferner ein zweites Wärmeabführungsglied (14) umfasst, das zweite Wärmeabführungsglied (14) zwischen dem ersten Wärmeabführungsglied (13) und dem Gehäuse (11) angeordnet ist und das zweite Wärmeabführungsglied (14) dazu ausgestaltet ist, die Wärme von dem ersten Wärmeabführungsglied (13) zu dem Gehäuse zu lenken.

5. Narkosegerät nach Anspruch 4, **dadurch gekennzeichnet, dass** die strahlenförmigen Rippen (132) jeweils ein erstes Ende und ein zweites Ende umfassen, das erste Ende mit dem Verbindungsabschnitt (131) verbunden ist, die zweiten Enden mindestens einiger der strahlenförmigen Rippen (132) an eingeschlossenen Winkeln zu den strahlenförmigen Rippen (132) mit Kontaktabschnitten (1325) versehen sind, eine Vielzahl von Kontaktabschnitten (1325) in derselben Ebene angeordnet sind oder eine Vielzahl von Kontaktabschnitten (1325) zum Bilden einer Kontaktplatte verbunden sind und das zweite Wärmeabstrahlungsglied (14) zwischen den Kontaktabschnitten (1325) und dem Gehäuse (11) angeordnet ist.

6. Narkosegerät nach Anspruch 4, **dadurch gekennzeichnet, dass** das Gehäuse (11) aus einem Metallmaterial hergestellt ist oder
das Gehäuse (11) aus wärmeleitendem Kunststoff hergestellt ist oder
eine Seitenwand des Gehäuses (11), die mit dem zweiten Wärmeabführungsglied (14) in Kontakt ist, aus einem Metallmaterial oder aus wärmeleitendem Kunststoff hergestellt ist.

7. Narkosegerät nach Ansprüchen 1 - 6, **dadurch gekennzeichnet, dass** der erste Lufteinlass (112) in der Nähe des Motors (123) angeordnet ist, so dass das meiste des von dem ersten Lufteinlass (112) in den ersten inneren Hohlraum (111) eintretenden Gases über eine Oberfläche des Motors (123) strömt, um die beim Betrieb des Motors (123) erzeugte Wärme wegzutransportieren.

8. Narkosegerät nach Ansprüchen 1 - 7, **dadurch gekennzeichnet, dass** die Schnecke (121) einen zweiten inneren Hohlraum (1211), einen zweiten Lufteinlass (1212), der mit dem zweiten inneren Hohlraum (1211) kommuniziert, und einen zweiten Luftauslass (1213), der mit dem zweiten inneren Hohlraum (1211) kommuniziert, umfasst, das Laufrad in dem zweiten inneren Hohlraum (1211) angeordnet ist, der zweite Luftauslass (1213) mit dem ersten Luftauslass (113) kommuniziert und der zweite Lufteinlass (1212) nahe dem ersten Lufteinlass (112) vorgesehen ist.

9. Narkosegerät nach Ansprüchen 1 - 8, **dadurch gekennzeichnet, dass** die Gebläsebaugruppe (10) ferner ein erstes elastisches Stützglied (15) und ein zweites elastisches Stützglied (16) umfasst, das Gehäuse (11) eine obere Platte (114) und eine der oberen Platte (114) gegenüberliegende untere Platte (115) umfasst, das erste elastische Stützglied (15) zwischen der oberen Platte (114) und der Oberseite des Gebläses liegt und das zweite elastische Stützglied (16) zwischen der unteren Platte (115) und der Unterseite des Gebläses liegt.

10. Narkosegerät nach Anspruch 9, **dadurch gekennzeichnet, dass** das zweite elastische Stützglied (16) um den zweiten Lufteinlass (1212) herum angeordnet ist und eine Seitenwand des zweiten elastischen Stützglieds (16) mit einem Spalt versehen ist, der mit dem zweiten Lufteinlass (1212) kommuniziert.

11. Narkosegerät nach Anspruch 4, **dadurch gekennzeichnet, dass** das erste Wärmeabführungsglied (13) mittels des zweiten Wärmeabführungsglieds (14) mit dem Gehäuse (11) verbunden ist und das erste Wärmeabführungsglied (13) und/oder das zweite Wärmeabführungsglied (14) aus einem flexiblen Material hergestellt ist.

12. Narkosegerät nach Anspruch 1, **dadurch gekennzeichnet, dass** das Narkosegerät ferner Folgendes umfasst:
das Gehäuse (11), das ferner einen dritten inneren Hohlraum (118) und einen dritten Lufteinlass (119) umfasst, der mit dem dritten inneren Hohlraum (118) kommuniziert, wobei der dritte innere Hohlraum (118) über den ersten Lufteinlass (112) mit dem ersten inneren Hohlraum (111) kommuniziert,
das Gebläse, das in dem ersten inneren Hohlraum (111) angeordnet und dazu ausgestaltet ist, Luft von dem dritten Lufteinlass (119) in den ersten inneren Hohlraum (111) zu treiben und die Luft aus dem ersten Luftauslass (113) herauszublasen, und
eine erste Geräuschreduzierungsbaugruppe (17), die in dem dritten Hohlraum (118) angeordnet und zur Reduzierung des Gebläsegeräuschs von dem dritten Lufteinlass (119) ausgestaltet ist.

13. Narkosegerät nach Anspruch 12, **dadurch gekennzeichnet, dass** die erste Geräuschreduzierungsbaugruppe (17) mit einem Geräuschreduzierungskanal (171) versehen ist, wobei ein Ende des Geräuschreduzierungskanals (171) mit dem ersten Lufteinlass kommuniziert und das andere Ende des Geräuschreduzierungskanals (171) mit dem dritten Lufteinlass (119) kommuniziert.

14. Narkosegerät nach Ansprüchen 1 - 13, **dadurch gekennzeichnet, dass** der erste Lufteinlass dem Motor (123) gegenüberliegt.

15. Narkosegerät nach Anspruch 14, **dadurch gekennzeichnet, dass** der erste Lufteinlass (112) dem Motor (123) gegenüberliegt, und zwar (a) dass der erste Lufteinlass (112) und der Motor (123) einander in einer Lufteinlassrichtung des ersten Lufteinlasses (112) direkt gegenüberliegen oder (b) dass sich der erste Lufteinlass (112) und der Motor (123) einander in der Lufteinlassrichtung des ersten Lufteinlasses (112) teilweise überlappen.

## Revendications

1. Machine d'anesthésie, la machine d'anesthésie comprenant une branche de gaz d'entraînement (100), une branche de gaz frais (200) et un circuit respiratoire (300), dans laquelle la branche de gaz frais (200) est conçue pour administrer du gaz frais avec du gaz anesthésique dans le circuit respiratoire (300), la branche de gaz d'entraînement (100) est conçue pour pousser le gaz frais provenant du circuit respiratoire (300) vers un patient, la branche de gaz d'entraînement (100) comprend un ensemble ventilateur (10), **caractérisée en ce que**
l'ensemble ventilateur (10) comprend un boîtier (11), un ventilateur (12) et un premier élément de dissipation de chaleur (13),
le boîtier (11) comprend une première cavité interne (111), une première entrée d'air (112) communiquant avec la première cavité interne (111), et une première sortie d'air (113) communiquant avec la première cavité interne (111),
le ventilateur (12) est disposé dans la première cavité interne (111) et conçu pour entraîner l'air provenant de la première entrée d'air (112) dans la première cavité interne (111) et souffler l'air hors de la première sortie d'air (113), et
le ventilateur (12) comprend :
une volute (121) ;
une hélice, montée rotative dans la volute (121) ; et
un moteur (123), monté dans la volute (121) et raccordé à l'hélice, et conçu pour entraîner l'hélice en rotation ;
dans laquelle le premier élément de dissipation de chaleur (13) est monté sur le moteur (123) et raccordé au boîtier (11), et le premier élément de dissipation de chaleur (13) est conçu pour conduire la chaleur générée par le moteur (123) vers le boîtier (11).

2. Machine d'anesthésie selon la revendication 1, **caractérisée en ce que** le premier élément de dissipation de chaleur (13) comprend :
une partie de raccordement (131) raccordée au moteur (123) ; et
des ailettes rayonnantes (132) raccordées à la partie de raccordement (131) pour dilater une zone de dissipation de chaleur.

3. Machine d'anesthésie selon la revendication 2, **caractérisée en ce que** la partie de raccordement (131) est en forme d'anneau, la partie de raccordement est manchonnée sur le moteur (123), une pluralité d'ailettes rayonnantes est fournie, et la pluralité d'ailettes rayonnantes (132) est espacée autour d'une paroi latérale externe de la partie de raccordement.

4. Machine d'anesthésie selon la revendication 3, **caractérisée en ce que** l'ensemble ventilateur (10) comprend en outre un second élément de dissipation de chaleur (14), le second élément de dissipation de chaleur (14) est disposé entre le premier élément de dissipation de chaleur (13) et le boîtier (11), et le second élément de dissipation de chaleur (14) est conçu pour diriger la chaleur provenant du premier élément de dissipation de chaleur (13) vers le boîtier.

5. Machine d'anesthésie selon la revendication 4, **caractérisée en ce que** les ailettes rayonnantes (132) comprennent chacune une première extrémité et une seconde extrémité, la première extrémité est raccordée à la partie de raccordement (131), les secondes extrémités d'au moins certaines des ailettes rayonnantes (132) sont pourvues de parties de contact (1325) à des angles inclus par rapport aux ailettes rayonnantes (132), une pluralité de parties de contact (1325) est disposée dans le même plan, ou une pluralité de parties de contact (1325) est raccordée pour former une plaque de contact, et le second élément de rayonnement thermique (14) est intercalé entre les parties de contact (1325) et le boîtier (11).

6. Machine d'anesthésie selon la revendication 4, **caractérisée en ce que** le boîtier (11) est en matériau métallique ; ou
le boîtier (11) est en plastique thermoconducteur ; ou
une paroi latérale du boîtier (11) qui est en contact avec le second élément de dissipation de chaleur (14) est en matériau métallique ou plastique thermoconducteur.

7. Machine d'anesthésie selon les revendications 1 à 6, **caractérisée en ce que** la première entrée d'air (112) est située à proximité du moteur (123), de sorte que la majeure partie du gaz pénétrant dans la première cavité interne (111) à partir de la première entrée d'air (112) s'écoule sur une surface du moteur (123) pour emporter la chaleur générée lors du fonctionnement du moteur (123).

8. Machine d'anesthésie selon les revendications 1 à 7, **caractérisée en ce que** la volute (121) comprend une deuxième cavité interne (1211), une deuxième entrée d'air (1212) communiquant avec la deuxième cavité interne (1211), et une deuxième sortie d'air (1213) communiquant avec la deuxième cavité interne (1211), l'hélice est disposée dans la deuxième cavité interne (1211), la deuxième sortie d'air (1213) communique avec la première sortie d'air (113), et la deuxième entrée d'air (1212) est située à proximité de la première entrée d'air (112).

9. Machine d'anesthésie selon les revendications 1 à 8, **caractérisée en ce que** l'ensemble ventilateur (10) comprend en outre un premier élément de support élastique (15) et un second élément de support élastique (16), le boîtier (11) comprend une plaque supérieure (114) et une plaque inférieure (115) en regard de la plaque supérieure (114), le premier élément de support élastique (15) est intercalé entre la plaque supérieure (114) et le haut du ventilateur, et le second élément de support élastique (16) est intercalé entre la plaque inférieure (115) et le bas du ventilateur.

10. Machine d'anesthésie selon la revendication 9, **caractérisée en ce que** le second élément de support élastique (16) est disposé autour de la deuxième entrée d'air (1212), et qu'une paroi latérale du second élément de support élastique (16) est pourvue d'un espace communiquant avec la deuxième entrée d'air (1212).

11. Machine d'anesthésie selon la revendication 4, **caractérisée en ce que** le premier élément de dissipation de chaleur (13) est raccordé au boîtier (11) par le second élément de dissipation de chaleur (14), et qu'au moins l'un du premier élément de dissipation de chaleur (13) et du second élément de dissipation de chaleur (14) est en matériau flexible.

12. Machine d'anesthésie selon la revendication 1, **caractérisée en ce que** la machine d'anesthésie comprend en outre
le boîtier (11) comprenant en outre une troisième cavité interne (118), et une troisième entrée d'air (119) communiquant avec la troisième cavité interne (118), la troisième cavité interne (118) communiquant avec la première cavité interne (111) par l'intermédiaire de la première entrée d'air (112) ;
le ventilateur, disposé dans la première cavité interne (111) et conçu pour entraîner l'air depuis la troisième entrée d'air (119) dans la première cavité interne (111) et souffler l'air hors de la première sortie d'air (113) ; et
un premier ensemble de réduction du bruit (17), disposé dans la troisième cavité interne (118) et conçu pour réduire le bruit du ventilateur provenant de la troisième entrée d'air (119).

13. Machine d'anesthésie selon la revendication 12, **caractérisée en ce que** le premier ensemble de réduction du bruit (17) est pourvu d'un canal de réduction du bruit (171), une extrémité du canal de réduction du bruit (171) communiquant avec la première entrée d'air et l'autre extrémité du canal de réduction du bruit (171) communiquant avec la troisième entrée d'air (119).

14. Machine d'anesthésie selon les revendications 1 à 13, **caractérisée en ce que** la première entrée d'air est en regard du moteur (123).

15. Machine d'anesthésie selon la revendication 14, **caractérisée en ce que** la première entrée d'air (112) est en regard du moteur (123), y compris (a) la première entrée d'air (112) et le moteur (123) étant directement en regard l'un de l'autre dans une direction d'admission d'air de la première entrée d'air (112) ou (b) la première entrée d'air (112) et le moteur (123) se chevauchant partiellement dans la direction d'admission d'air de la première entrée d'air (112).
